(19) Europäisches Patentamt
European Patent Office
Office européen des brevets

(11) **EP 3 559 661 B1**

(12) **FASCICULE DE BREVET EUROPEEN**

(45) Date de publication et mention
de la délivrance du brevet:
**07.04.2021 Bulletin 2021/14**

(21) Numéro de dépôt: **17821980.4**

(22) Date de dépôt: **12.12.2017**

(51) Int Cl.:
***G01N 33/38*** *(2006.01)*

(86) Numéro de dépôt international:
**PCT/FR2017/053512**

(87) Numéro de publication internationale:
**WO 2018/115636 (28.06.2018 Gazette 2018/26)**

(54) **CELLULE DE MESURE ET PROCEDE DE MESURE ASSOCIE**

MESSZELLE UND ZUGEHÖRIGES MESSVERFAHREN

MEASUREMENT CELL AND ASSOCIATED MEASUREMENT METHOD

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **23.12.2016 FR 1663366**

(43) Date de publication de la demande:
**30.10.2019 Bulletin 2019/44**

(73) Titulaire: **Curis International
69250 Curis-Au-Mont-D'or (FR)**

(72) Inventeurs:
• **BOIS, Axel-Pierre
69250 Curis-au-Mont-D'or (FR)**
• **VU, Manh-Huyen
69003 Lyon (FR)**

(74) Mandataire: **Cabinet Laurent & Charras
Le Contemporain
50 Chemin de la Bruyère
69574 Dardilly Cedex (FR)**

(56) Documents cités:
**FR-A1- 2 965 925     US-A- 2 217 278**

**Description**

**DOMAINE TECHNIQUE**

L'invention concerne une cellule de mesure d'au moins une propriété physique, chimique ou mécanique d'un matériau durcissable ainsi que le procédé de mesure associé.

L'invention trouve une application particulièrement avantageuse pour mesurer les propriétés mécaniques d'un ciment de sorte à modéliser le comportement d'une gaine de ciment ou d'un bouchon de ciment d'un puits pétrolier.

**TECHNIQUES ANTERIEURES**

La cimentation d'un cuvelage d'un puits pétrolier consiste à placer une gaine de ciment dans l'annulaire entre un cuvelage et la paroi du trou, le trou pouvant être constitué par un autre cuvelage ou par de la roche. Cette gaine de ciment a un rôle primordial dans la stabilité et l'isolation des puits pétroliers.

Le bouchage d'un puits d'hydrocarbures vise à rétablir l'intégrité naturelle des formations qui ont été pénétrées par le forage. Pour obturer un puits, il est classique de positionner des bouchons de ciment au droit de certaines formations géologiques afin d'isoler les réservoirs.

La gaine et le bouchon de ciment sont réalisés par pompage d'un coulis de ciment fabriqué à partir de ciment, d'eau et d'adjuvants. Ce coulis de ciment est à l'état liquide lorsqu'il est pompé. L'hydratation des particules de ciment se produit par dissolution et précipitation d'ions, conduisant le coulis liquide vers un état solide, caractérisé par l'existence d'un squelette poreux.

La gaine et le bouchon de ciment sont exposés à diverses sollicitations mécaniques et thermiques durant la vie du puits provenant d'opérations menées dans le puits (tests en pression, changement de boue, stimulations froides et chaudes, ...) ou de phénomènes prenant naissance directement dans le sous-sol (compaction du réservoir, séismes, ...) et ce jusqu'à son abandon, voire au-delà.

Ces sollicitations peuvent endommager le matériau constitutif de la gaine ou du bouchon de ciment, dégrader ses propriétés mécaniques et sa perméabilité et, par conséquent, modifier sa contribution dans la stabilité et l'étanchéité du puits. La connaissance du comportement de ce ciment dans les conditions de puits et de son évolution au cours du temps est essentielle pour l'analyse du fonctionnement du puits pendant l'exploitation et aussi pour garantir son étanchéité pour le stockage de gaz, en particulier les gaz à effet de serre ($CO_2$, par exemple).

Les caractéristiques du coulis de ciment sont déterminées en fonction d'un grand nombre de paramètres dont l'état des terrains au droit de la gaine ou du bouchon de ciment (température, pression de pore, état de contraintes), les caractéristiques du puits (densité des fluides de forages, stabilité du puits, déviation, diamètre du trou foré), la localisation de la gaine ou du bouchon de ciment dans le puits, la technique de pompage utilisée, les sollicitations auxquelles la gaine ou le bouchon de ciment seront soumis, les propriétés mécaniques recherchées du ciment,

Ainsi, indépendamment de l'application envisagée, il est important de connaître avec précision les propriétés physiques, chimiques ou mécaniques du ciment ainsi que leur évolution durant son hydratation. Pour ce faire, il est connu de procéder à des essais statiques en réalisant la prise d'un échantillon de ciment dans un moule parallélépipédique ou cylindrique.

Lorsque l'échantillon est solide, il est extrait du moule et les propriétés physiques, chimiques ou mécaniques de cet échantillon sont testées.

Par exemple, pour les propriétés mécaniques, un test en traction ou en compression est réalisé selon un ou plusieurs axes.

Cependant, ce type de procédé est complexe et long à mettre en œuvre car l'échantillon passe par deux dispositifs, à savoir un premier dispositif pour réaliser la solidification et un second dispositif pour réaliser les mesures. En outre, l'échantillon est préférentiellement réalisé dans les conditions de fond d'un puits pétrolier, par exemple avec une température et une pression importante. Lorsque l'échantillon est extrait du moule, les changements de température et de pression peuvent dégrader les propriétés physiques, chimiques ou mécaniques de l'échantillon. Il s'ensuit que ce type de procédé ne permet pas de mesurer précisément les propriétés physiques, chimiques ou mécaniques d'un ciment durci dans les conditions de fond d'un puits pétrolier.

De plus, il est également recherché de connaître l'évolution des propriétés physiques, chimiques ou mécaniques des ciments au cours de leur hydratation afin de pouvoir calculer l'état de contraintes dans le ciment une fois durci. Ceci ne peut pas être réalisé avec les essais statiques conventionnels car il faut pouvoir tester les échantillons avant qu'ils ne soient durcis.

Pour remédier à ces problèmes, il existe plusieurs dispositifs qui proposent des cellules de mesure aptes à réaliser à la fois la solidification du coulis dans un moule et certaines mesures sans déplacer l'échantillon.

Par exemple, la demande de brevet internationale N° WO 2012/049620 décrit une enceinte pourvue d'un plateau supérieur et d'un plateau inférieur entre lesquelles sont montés deux demi-cylindres mobiles en translation.

Pour réaliser la prise et la caractérisation d'un échantillon de ciment, une membrane souple est disposée à l'intérieur des deux demi-cylindres.

Les deux demi-cylindres sont ensuite déplacés autour de la membrane de sorte à former une coque cylindrique autour de la membrane. Le ciment est alors introduit dans la membrane. Dans cette phase durant laquelle le ciment est liquide, le ciment est retenu par l'enceinte solide autour de la membrane. La membrane permet d'empêcher le coulis de ciment d'adhérer aux deux demi-cylindres et permet d'appliquer par la suite une pression de confinement sans que le fluide utilisé pour cela ne pénètre les pores du ciment.

Lors de la solidification, l'échantillon est placé dans les conditions de fond d'un puits pétrolier en termes de pression et température. Pour ce faire, une pression est appliquée sur le ciment placé dans la membrane par déplacement du plateau inférieur et/ou supérieur. Une pression est également appliquée dans l'enceinte par injection d'un fluide autour des deux demi-cylindres pour simuler les pressions subies par le ciment dans les conditions de fond d'un puits pétrolier.

Après un temps prédéterminé, des mesures sont effectuées sur cet échantillon tout en maintenant les conditions de fond en pression et en température dans l'enceinte.

Pour ce faire, les deux demi-cylindres possèdent deux demi-alésages disposés sensiblement à mi-hauteur des demi-cylindres. Lorsque les deux demi-cylindres sont déplacés autour de la membrane, les deux demi-alésages coopèrent pour former un seul alésage circulaire. Un capteur est positionné dans l'alésage jusqu'à atteindre la membrane et le capteur mesure les variations de diamètre de l'échantillon. D'autres mesures sont réalisées sur le ciment durci après que les deux demi-cylindres aient été déplacés en s'éloignant de la membrane.

Pour finir, le fluide est purgé de l'enceinte et le capteur ainsi que les deux demi-cylindres sont déplacés en s'éloignant de la membrane. L'échantillon et la membrane peuvent ensuite être extraits de l'enceinte et un autre test peut être effectué en positionnant une nouvelle membrane souple dans l'enceinte.

Ce dispositif est particulièrement performant car il permet d'effectuer plusieurs mesures en suivant le protocole standard des essais triaxiaux. Son principal inconvénient est de nécessiter la présence de pièces mobiles, les deux demi-cylindres, ce qui entraine des problèmes d'encombrement et des problèmes opérationnels tels que le risque que les demi-cylindres ne se referment pas complètement engendrant une fuite de ciment liquide, ou ne s'ouvre pas complètement, faussant ainsi les mesures réalisées ultérieurement. Ainsi les mesures de déformations radiales doivent être réalisées à l'aide de capteurs en position radiale et il est nécessaire, à la fin de chaque test, de purger l'enceinte de son huile. En conséquence, la préparation et le démontage d'un essai prennent plusieurs heures.

Le problème technique de l'invention consiste à améliorer la prise de mesure d'au moins une propriété physique, chimique ou mécanique d'un matériau durcissable.

## EXPOSE DE L'INVENTION

Pour résoudre ce problème technique, l'invention propose une nouvelle cellule de mesure comportant une membrane associée à un ensemble de fils métalliques assurant la rigidification de la membrane, ce qui est requis lors de la solidification du matériau durcissable.

A cet effet, selon un premier aspect, l'invention concerne une cellule de mesure d'au moins une propriété physique, chimique ou mécanique d'un matériau durcissable, ladite cellule comportant :

- une enceinte ;
- une membrane souple disposée dans ladite enceinte de sorte à contenir ledit matériau durcissable ; et
- des moyens de rigidification de ladite membrane configurés pour prendre deux états alternatifs :

  ◦ un état de rigidité dans lequel lesdits moyens de rigidification résistent à l'expansion dudit matériau durcissable lors de la solidification ; et
  ◦ un état de flexibilité dans lequel lesdits moyens de rigidification exercent une contrainte inférieure à celle exercée dans l'état de rigidité permettant de mesurer au moins une propriété physique, chimique ou mécanique dudit matériau durcissable et d'extraire ledit matériau durcissable de ladite enceinte ;

- lesdits moyens de rigidification étant réalisés par un ensemble de fils métalliques reliés sur un fermoir configuré pour modifier la rigidité exercée par lesdits fils métalliques.

L'invention permet ainsi de limiter l'encombrement de l'enceinte en intégrant les moyens de rigidification au niveau de la membrane. En outre, la suppression des deux demi-sphères permet de faciliter l'intégration du ou des capteurs autour de la membrane. En particulier, au lieu d'utiliser un capteur orienté dans la direction radiale de l'échantillon, il est désormais possible d'utiliser une chainette disposée autour de la membrane pour mesurer les variations de circonférence de l'échantillon.

En outre, après les mesures, l'échantillon solidifié peut ainsi être extrait de l'enceinte sans entrainer la membrane car les moyens de rigidification sont dans l'état de flexibilité. Il n'est donc plus nécessaire de remplacer la membrane pour chaque test. Il s'ensuit que le fluide autour de la membrane peut également être conservé entre deux tests successifs ce qui réduit le temps de cycle de chaque test en supprimant les étapes de pompage et de soutirage du fluide autour de la membrane.

Selon un mode de réalisation, lesdits fils métalliques sont disposés de manière circonférentielle à plusieurs hauteurs de ladite membrane, les extrémités de chaque fil métallique étant fixées sur ledit fermoir. Ce mode de réalisation permet d'appliquer une contre-pression radiale sur l'échantillon lors de la solidification. La pression appliquée sur la membrane est sensiblement équivalente à la pression appliquée par les deux demi-cylindres de l'art antérieur tout en étant plus simple à mettre en œuvre et plus compact.

Selon un mode de réalisation, le nombre *n* de fils métalliques respecte la condition suivante :

$$\frac{L}{2r+e} \leq n \leq \frac{mL}{2r};$$

avec *L* correspondant à la hauteur d'une membrane cylindrique (15), *r* au rayon ou a la demi-largeur des fils métalliques, *m* au nombre de nappes de fils et *e* à l'épaisseur de la membrane.

Selon un mode de réalisation, ladite enceinte comporte au moins un capteur de déplacement radial disposé de manière circonférentielle autour de ladite membrane entre deux fils métalliques. Ce mode de réalisation permet de disposer un ou plusieurs capteurs circonférentiels autour de la membrane, limitant ainsi l'encombrement et améliorant la précision des mesures. En variante ou en complément, un capteur axial peut être utilisé.

Selon un mode de réalisation, ladite membrane est réalisée avec une âme en élastomère. De préférence, ladite membrane est réalisée avec une âme en polytétrafluoroéthylène, tel que commercialisé sous la dénomination Téflon® ou en caoutchouc fluorocarbone tel que commercialisé sous la dénomination Viton®. Ce mode de réalisation permet de garantir la souplesse de la membrane tout en limitant l'accrochage du matériau durcissable sur la membrane.

En outre, le Teflon® ou le Viton® sont des matériaux aptes à supporter l'intégration des moyens de rigidification, tels que des fils métalliques.

Selon un mode de réalisation, ladite enceinte est étanche à un fluide mis sous pression de sorte à permettre l'application d'une pression autour de ladite membrane. Ce mode de réalisation vise à appliquer une pression sur le matériau durcissable lors de la solidification, par exemple pour simuler les conditions de fond d'un puits pétrolier, ou lors de la réalisation d'essais triaxiaux, par exemple pour mesurer des propriétés mécaniques sous confinement.

Selon un mode de réalisation, ladite enceinte intègre des moyens de chauffage de sorte à imposer une température autour de ladite membrane. Ce mode de réalisation vise à appliquer une température sur le matériau durcissable lors de la solidification, par exemple pour simuler les conditions de fond d'un puits pétrolier ou lors de la réalisation d'essais triaxiaux, par exemple pour mesurer des propriétés mécaniques.

Selon un mode de réalisation, ladite membrane est disposée entre un plateau inférieur et un plateau supérieur disposés au niveau de deux ouvertures de l'enceinte, au moins un plateau étant mobile en translation de sorte à compresser ledit matériau durcissable lors de la solidification, lors de la réalisation de tests et/ou de sorte à extraire ledit matériau durcissable de l'enceinte.

Ce mode de réalisation vise à appliquer une pression axiale sur le matériau durcissable lors de la solidification, par exemple pour simuler les conditions de fond d'un puits pétrolier. Ce mode de réalisation permet également d'appliquer une contrainte axiale lors de la réalisation d'essais triaxiaux, par exemple pour mesurer des propriétés mécaniques.

Selon un second aspect, l'invention concerne un procédé de mesure d'au moins une propriété physique, chimique ou mécanique d'un matériau durcissable lors de la solidification ou après au moyen d'une cellule de mesure selon le premier aspect de l'invention.

Ledit procédé comporte les étapes consistant à :

- mettre lesdits moyens de rigidification dans ledit état de rigidité ;
- verser ledit matériau durcissable sous forme liquide dans ladite membrane ;
- fermer ladite enceinte ;
- solidifier ledit matériau durcissable ;
- lorsque l'état de solidification requis est atteint, mettre lesdits moyens de rigidification dans ledit état de flexibilité ;
- mesurer au moins une propriété physique, chimique ou mécanique dudit matériau durcissable ; et
- extraire ledit matériau durcissable de ladite enceinte.

Ce deuxième aspect de l'invention permet ainsi de mesurer au moins une propriété physique, chimique ou mécanique d'un matériau durcissable sans déplacer le matériau hors de l'enceinte.

Selon un mode de réalisation, lorsque ladite cellule de mesure intègre une enceinte étanche, la solidification dudit matériau durcissable est réalisée en appliquant une rampe de pression dans ladite enceinte.

Selon un mode de réalisation, lorsque ladite cellule de mesure intègre des moyens de chauffage, la solidification dudit matériau durcissable est réalisée en appliquant une rampe de température dans ladite enceinte.

Selon un mode de réalisation, lorsque ladite cellule de mesure intègre un plateau mobile, la solidification dudit matériau durcissable est réalisée en appliquant une rampe de contrainte axiale sur ledit matériau durcissable.

Selon un mode de réalisation, l'étape de mesure d'au moins une propriété physique, chimique ou mécanique dudit matériau durcissable consiste à mesurer les déformations axiale et radiale, et/ou la pression de pore, et/ou la vitesse des ondes soniques de compression et de cisaillement, et/ou la résistivité.

A partir d'une partie ou de l'ensemble de ces mesures, un grand nombre de propriétés physiques, chimiques ou mécaniques du matériau durcissable peuvent être obtenues. Par exemple, à partir du module de Young et du coefficient de Poisson drainés, il est possible de retrouver le module de cisaillement. A partir de la vitesse des ondes de cisaillement, il est possible de déterminer le seuil de percolation du coulis de ciment.

Selon un mode de réalisation, suite à l'extraction dudit matériau durcissable de ladite enceinte, ledit procédé comporte une étape consistant à analyser ledit matériau durcissable. Ce mode de réalisation permet de réaliser d'autres analyses sur le matériau durcissable, non réalisables dans l'enceinte. Par exemple, un simple examen visuel peut être réalisé ou un examen plus complexe tel qu'une tomographie par rayons X.

## DESCRIPTION SOMMAIRE DES FIGURES

La manière de réaliser l'invention, ainsi que les avantages qui en découlent ressortiront bien de la description du mode de réalisation qui suit, à l'appui des figures annexées dans lesquelles :

- la figure 1 est une vue en coupe d'une cellule de mesure selon un premier mode de réalisation de l'invention ; et
- la figure 2 est une vue en coupe d'une cellule de mesure selon un second mode de réalisation de l'invention.

## MANIERES DE REALISER L'INVENTION

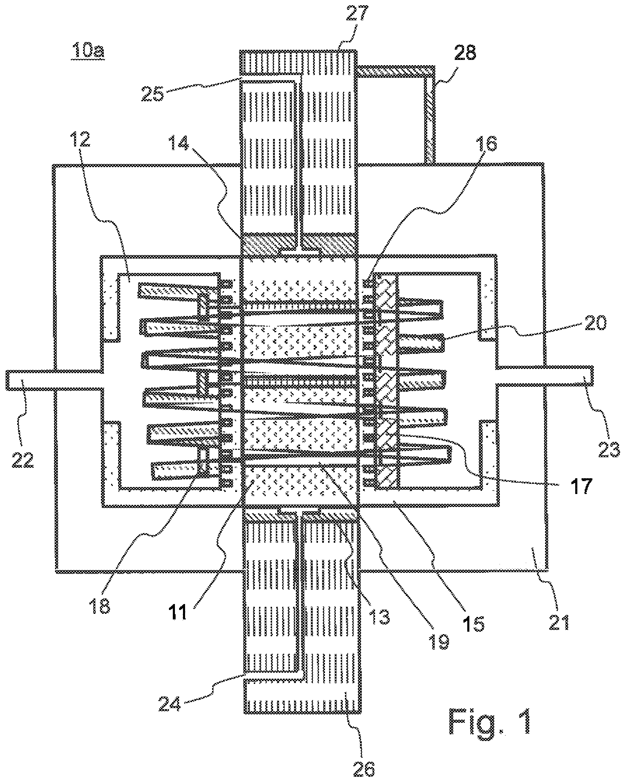

La figure 1 illustre une cellule de mesure **10a** comportant une enceinte **12** destinée à solidifier et mesurer au moins une propriété physique, chimique ou mécanique d'un matériau durcissable **11**.

Le matériau durcissable **11** peut être un coulis de ciment mais d'autres matériaux peuvent être utilisés sans changer l'invention, par exemple les résines ou les colles. Dans l'exemple de la figure 1, le matériau durcissable **11** est formé sous la forme d'un cylindre mais d'autres formes peuvent être mises en œuvre sans changer l'invention.

L'enceinte **12** prend la forme d'une chambre cylindrique ménagée dans un bâti **21** en métal, par exemple en acier ou en aluminium. Le bâti **21** présente une ouverture supérieure et une ouverture inférieure. Les ouvertures sont obturées par des plateaux **13**, **14** mobiles sous l'effet d'un ou deux pistons **26**, **27**. Le matériau durcissable **11** est disposé entre les deux plateaux **13**, **14**. Un canal fluidique **24** traverse le piston inférieur **26** et le plateau inférieur **13** pour déboucher dans une chambre du plateau inférieur **13** de sorte à appliquer une pression de pores à partir de l'extrémité inférieure du matériau durcissable **11**.

De la même manière, un canal fluidique **25** traverse le piston supérieur **27** et le plateau supérieur **14** pour déboucher dans une chambre du plateau supérieur **14** de sorte à appliquer une pression de pores à partir de l'extrémité supérieure du matériau durcissable **11**. Le matériau durcissable **11** est également contraint radialement par une membrane **15** déformable associée à un ensemble des fils métalliques **16** disposés radialement autour du matériau durcissable **11**. La membrane **15** est préférentiellement réalisée en élastomère, par exemple en Teflon® ou en Viton®.

Tel qu'illustré sur la figure 1, les fils métalliques **16** peuvent être noyés sur le bord externe de la membrane **15** autour du matériau durcissable **11**. Les fils métalliques **16** sont disposés à plusieurs hauteurs de la membrane **15**.

En variante, les fils métalliques **16** peuvent entourer la membrane **15**. En variante, les fils métalliques **16** peuvent former une grille métallique. En variante, les fils métalliques **16** peuvent s'étendre longitudinalement par rapport au matériau durcissable **11**.

Les extrémités des fils métalliques **16** sont fixées sur les deux faces d'un fermoir **17**, contrôlé à distance. Ce fermoir **17** peut prendre deux positions : une position fermée et une position ouverte.

Dans la position fermée, une tension est exercée sur les fils métalliques **16** de sorte à rigidifier la membrane **15** et contenir radialement le matériau durcissable **11**. Dans la position ouverte, les fils métalliques **16** sont dans un état de flexibilité.

La dimension et le nombre des fils **16** sont adaptés pour éviter l'extrusion de la membrane **15** si la pression à l'intérieure est supérieure à la pression à l'extérieure de la membrane **15**. Pour ce faire, il est possible de modéliser les contraintes subies par les fils **16** pour une membrane **15** de diamètres intérieur et extérieur $Ri$ et $Ro$ respectivement. La partie de la membrane **15** en contact avec le matériau durcissable **11**, est entourée par une nappe de $n$ fils d'acier de section circulaire de diamètre *r*. D'autres sections peuvent être utilisées sans changer l'invention, comme des sections carrés ou rectangulaires. La possibilité d'utiliser plusieurs nappes de fils est aussi possible. Les pressions extérieure et intérieure sur la membrane **15** sont notées respectivement *po* et *pi*.

La variation de la température dans la membrane **15** est considérée homogène. Les contraintes dans la membrane **15** sont calculées dans un premier temps en supposant qu'il n'y a pas de fils d'acier **16**. La contrainte tangentielle supportée par la membrane **15**, lorsqu'il n'y a pas de fil d'acier **16**, est donnée par :

$$\sigma_\theta = A + \frac{B}{r}$$

$$A = \frac{p_o R_o^2 - p_i R_i^2}{R_o^2 - R_i^2}$$

$$B = \frac{(p_o - p_i) \cdot R_o^2 R_i^2}{R_o^2 - R_i^2}$$

Ensuite, la contrainte dans les fils d'aciers **16** est obtenue en considérant l'équilibre de force entre la membrane **15** avec les contraintes calculées et les fils d'acier **16**. En considérant le système de la membrane **15** et les fils d'acier **16**, la force générée par la contrainte tangentielle de la membrane **15** est calculée par l'expression suivante :

$$F = L \cdot \int_{R_i}^{R_o} \left( A + \frac{B}{r} \right) \cdot dr$$

dans laquelle *L* correspond à la longueur de l'échantillon.

Cette expression peut être intégrée de la manière suivante :

$$F = L \cdot (R_o - R_i) \cdot \left( A + \frac{B}{R_o R_i} \right)$$

En remplaçant l'expression de A et B dans l'expression de F, on obtient :

$$F = \frac{L}{R_o + R_i} \cdot \left[ p_o R_o^2 - p_i R_i^2 + (p_o - p_i) R_o R_i \right]$$

La distribution de cette force sur *m* nappes de *n* fils d'acier **16** permet d'obtenir la contrainte suivante dans les fils **16** :

$$\sigma_w = \frac{F}{2nm \cdot \pi r^2}$$

Ainsi, lorsque la pression intérieure *pi* est beaucoup plus élevée que la pression extérieure *po*, la contrainte tangentielle *F* est négative et les fils **16** sont en traction. Lorsque la pression intérieure *pi* est légèrement plus élevée que la pression extérieure *po*, fils **16** sont en compression.

Pour garantir un fonctionnement correct de la cellule **10a**, le nombre de fils **16** doit respecter la condition suivante :

$$\frac{L}{2r + e} \leq n \leq \frac{mL}{2r}$$

Dans l'exemple de la figure 1, la membrane **15** présente une épaisseur *e* comprise entre 2 et 4 mm et les fils métalliques **16** présentent un diamètre compris entre 0.5 et 1 mm. Le matériau durcissable **11** présente une longueur comprise entre 65 et 80 mm et un diamètre compris entre 30 et 40 mm. Le nombre de fils **16** est préférentiellement compris entre 35 et 45 pour une simple nappe.

Ce système des fils **16** présente des aménagements afin de permettre le positionnement de capteurs **18** de mesure des déformations radiales. Ainsi, trois capteurs **18** de déplacement radial sont installés à trois hauteurs distinctes du matériau durcissable **11** à l'aide de trois chainettes **19** indéformables qui entourent la membrane **15**. Un capteur **28** de déplacement axial du plateau supérieur **14** est également disposé au-dessus du bâti **21**. En variante, la cellule **10a** peut également intégrer des capteurs pour mesurer la vitesse des ondes de compression et de cisaillement dans le matériau durcissable **11**, la résistivité du matériau **11**, ou tout autre paramètre physico- chemo-mécanique du matériau **11**.

Pour reproduire les conditions de pression, l'enceinte **12** est hermétique par l'utilisation d'une membrane **15** épousant, au moins partiellement les parois internes et externes de l'enceinte **12**.

Ainsi, lorsqu'une pression est appliquée dans l'enceinte **12**, le maintien de la membrane **15** contre le matériau **11** est assurée par la partie de la membrane **15** au contact du bord externe de l'enceinte **12**. La pression dans l'enceinte **12** est générée par un fluide de confinement injecté et contrôlé par deux conduits **22**, **23** traversant une partie centrale du bâti **21**.

Pour reproduire les conditions de température, un serpentin de chauffage **20** et/ou de refroidissement est installé dans la cellule **12**, immergé dans le fluide de confinement. Ce système a pour but de contrôler et/ou d'imposer efficacement la température du matériau **11**. De préférence, l'entrée et la sortie du système de chauffage/refroidissement sont placées au milieu du bâti **21**.

En variante ou en complément, une coque de chauffage externe peut également être disposée autour du bâti **21**.

Un exemple de mesure des propriétés physiques, chimiques ou mécaniques d'un bouchon de ciment est décrit ci-après en utilisant la cellule **10a** de la figure 1.

Dans une première étape, le plateau inférieur **13** est positionné au niveau de l'extrémité inférieure de l'enceinte **12**. La membrane **15** est rigidifiée par fermeture du fermoir **17**. Un coulis de ciment liquide est injecté dans la membrane **15** par l'ouverture supérieure de l'enceinte **12** jusqu'au niveau de l'ouverture supérieure permettant ainsi d'obtenir un échantillon ayant la forme d'un cylindre plein. En variante, un cylindre peut être inséré au centre du coulis de ciment de sorte à former un échantillon ayant la forme d'un cylindre creux. Pour finir cette étape de préparation du test, le plateau supérieur **14** est mis en place de sorte à fermer la chambre de confinement du ciment.

Dans une seconde étape, le piston supérieur **27** est mis en action pour déplacer le plateau supérieur **14** de sorte à appliquer une pression dans le ciment, pression qui est reprise par les fils métallique car le fermoir est en position fermée. De même, une variation de la pression confinement est appliquée dans l'enceinte **12** en fonction du temps par l'intermédiaire des conduits **22**, **23** et une variation de température prédéterminée est appliquée dans l'enceinte **12** en fonction du temps par l'intermédiaire des moyens de chauffage et/ou de refroidissement **20**.

Les mesures des propriétés mécaniques du ciment peuvent être réalisées lors de la solidification du ciment et/ou après la solidification. Ainsi, dans une troisième étape, pour effectuer des mesures après un niveau de solidification requis, la membrane **15** est détendue par ouverture du fermoir **17**.

Un essai triaxial est ensuite réalisé en contrôlant la contrainte axiale, la pression de confinement, la température, la pression de pore et la composition physico-chimique du fluide de pore. Les déformations axiale et radiale sont mesurées par les capteurs **18**, **28** et la pression de pore est obtenue par les canaux **24**, **25**. D'autres mesures peuvent être effectuées pour obtenir la vitesse des ondes soniques de compression et de cisaillement, la mesure de la résistivité ou la mesure de tout autre paramètre physico-chemo-mécanique du ciment ou de ses constituants.

Dans une quatrième étape, après que la contrainte axiale, la pression de confinement, la pression de pore et la température de l'échantillon ont été ramenées aux valeurs ambiantes, l'échantillon est extrait de l'enceinte **12** par retrait du piston supérieur **27** et déplacement de l'échantillon par poussée du piston inférieur **26**. L'échantillon est ainsi récupéré et un examen visuel ou un examen par tomographie aux rayons X peut être effectué hors de l'enceinte **12** alors qu'une autre mesure peut être réalisée en replaçant la cellule **10a** dans la première étape.

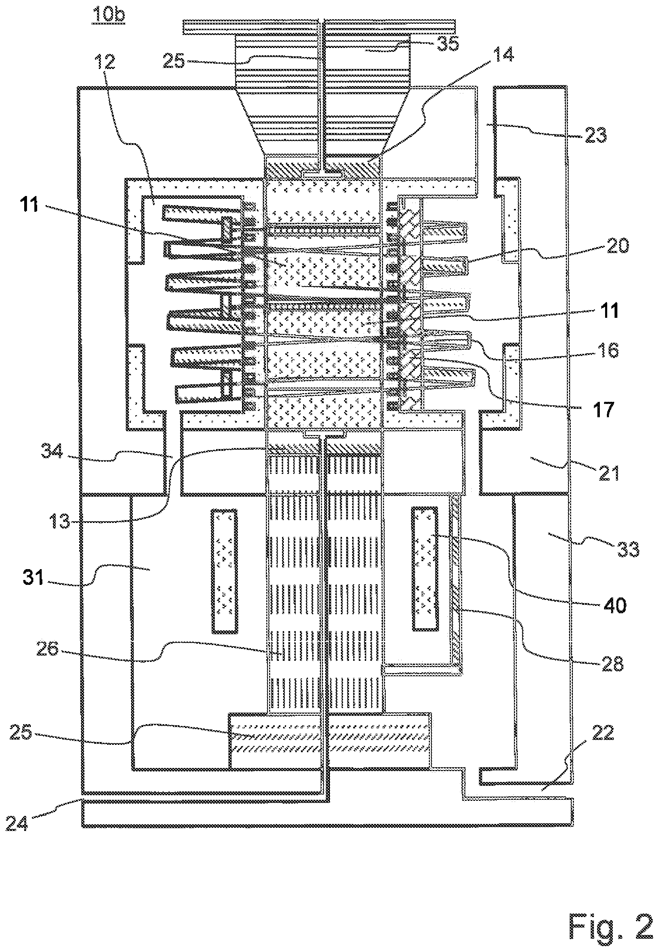

La figure 2 illustre une cellule **10b** fonctionnant selon le même principe que la cellule **10a** de la figure 1. Cette cellule **10b** intègre un bâti de chargement. Pour ce faire, la cellule **10b** est constituée de deux chambres : une enceinte **12** destinée à réaliser le confinement d'un matériau durcissable **11**, et une chambre de chargement **31**.

Le matériau durcissable **11** est solidifié et mesuré dans l'enceinte **12** alors que les vérins hydrauliques **25**, **26** et le capteur **28** de déplacement axial sont installés dans la chambre de chargement **31**.

La chambre de chargement **31** est connectée hydrauliquement avec l'enceinte **12** par des évidements **34** communiquant entre le bâti **21** formant l'enceinte **12** et le bâti **33** formant la chambre de chargement **31** de sorte à équilibrer la pression entre les deux chambres **21**, **31**.

Ce mode de réalisation permet d'augmenter la précision du capteur **28** de déplacement axial car il fonctionne à la même pression que l'enceinte **12**. De préférence, les deux chambres **12, 31** sont fixées entre elles par des boulons.

La chambre de chargement **31** comporte un vérin **25** à grande capacité de force, permettant de charger axialement le matériau **11** lors de la solidification ou des mesures, et un vérin **26** à grande capacité de déplacement, permettant d'extraire le matériau **11** de l'enceinte **12**. Le vérin supérieur est remplacé par un bouchon **35** fixé sur le bâti **21** après insertion du matériau durcissable **11** dans l'enceinte **12**.

L'entrée **22** du fluide de confinement est placée dans la chambre de chargement **31** alors que la sortie **23** du fluide de confinement est ménagée dans l'enceinte **12**. La pression de pore dans le matériau durcissable **11** est contrôlée par un générateur de pression à travers deux canaux **24**, **25**. Un premier canal **24** est ménagé dans le bâti **33** de la chambre de chargement **31**, les deux vérins **25**, **26** et le plateau inférieur **13** alors que le second canal est formé dans le bouchon **35** et le plateau supérieur **14**.

Pour reproduire les conditions de température, un serpentin de chauffage **20** et/ou de refroidissement est installé dans la cellule **12**, immergé dans le fluide de confinement. En outre, la chambre de chargement **31** présente des moyens de chauffage **40** configurés par maintenir un gradient de températures dans l'échantillon.

En variante ou en complément, une plaque chauffante peut être disposée sur le bouchon supérieur **35** de sorte à améliorer également le gradient de températures dans l'échantillon.

Le système de mesure du déplacement axial est constitué d'un capteur **28** de déplacement à haute précision soudé à un « patin magnétique ». Le patin magnétique est activé lorsque la course du capteur **28** de déplacement est atteinte.

Pendant les essais, le patin magnétique n'est pas activé et le capteur **28** de déplacement mesure le déplacement du vérin **26**. Lors de l'enlèvement du matériau durcissable **11**, le patin magnétique est activé et le vérin **26** peut se déplacer librement vers le haut sans endommager le capteur **28** de déplacement axial.

L'invention permet de réaliser la mesure des propriétés physiques, chimiques ou mécaniques d'un matériau durcissable **11** dans ses conditions d'utilisation pendant la phase d'hydratation jusqu'à la prise, voir au-delà, sans repasser par les conditions atmosphériques de pression et ambiantes de température.

La cellule **10b** correspond à une cellule triaxiale car les mesures sont effectuées sous variations de contraintes axiales en imposant une pression de confinement autour du matériau durcissable **11**. Ces mesures permettent d'obtenir les propriétés statiques de déformation et de rupture en conditions triaxiale durant ou après la prise, ainsi que les propriétés de conductivité hydrauliques.

**Revendications**

1.  Cellule de mesure (10a-10b) d'au moins une propriété physique, chimique ou mécanique d'un matériau durcissable (11), ladite cellule (10a-10b) comportant :

    - une enceinte (12) ;
    - une membrane souple (15) disposée dans ladite enceinte (12) de sorte à contenir ledit matériau durcissable (11) ; et
    - des moyens de rigidification (16, 17) de ladite membrane (15) configurés pour prendre deux états alternatifs :

        o un état de rigidité dans lequel lesdits moyens de rigidification (16, 17) résistent à l'expansion dudit matériau durcissable (11) lors de la solidification ; et
        o un état de flexibilité dans lequel lesdits moyens de rigidification (16, 17) exercent une contrainte inférieure à celle exercée dans l'état de rigidité permettant de mesurer au moins une propriété physique, chimique ou mécanique dudit matériau durcissable (11) et d'extraire ledit matériau durcissable (11) de ladite enceinte (12) ;

    **caractérisée en ce que** lesdits moyens de rigidification sont réalisés par un ensemble de fils métalliques (16) reliés sur un fermoir (17) configuré pour modifier la rigidité exercée par lesdits fils métalliques (16).

2.  Cellule de mesure selon la revendication 1, **dans laquelle** lesdits fils métalliques (16) sont disposés de manière circonférentielle à plusieurs hauteurs de ladite membrane (15), les extrémités de chaque fil métallique (16) étant fixées sur ledit fermoir (17).

3.  Cellule de mesure selon la revendication 1 ou 2, **dans laquelle** le nombre *n* de fils métalliques (16) respecte la condition suivante :

$$\frac{L}{2r+e} \leq n \leq \frac{mL}{2r};$$

avec $L$ correspondant à la hauteur d'une membrane cylindrique (15), $r$ au rayon ou a la demi-largeur des fils métalliques, m au nombre de nappes de fils et e à l'épaisseur de la membrane.

**4.** Cellule de mesure selon la revendication 2, ***dans laquelle*** ladite enceinte (12) comporte au moins un capteur (18) de déplacement radial disposé de manière circonférentielle autour de ladite membrane (15) entre deux fils métalliques (16).

**5.** Cellule de mesure selon l'une des revendications 1 à 4, ***dans laquelle*** ladite membrane (15) est réalisée avec une âme en élastomère.

**6.** Cellule de mesure selon l'une la revendication 5, ***dans laquelle*** ladite membrane (15) est réalisée avec une âme en polytétrafluoroéthylène, tel que commercialisé sous la dénomination Téflon® ou en caoutchouc fluorocarbone tel que commercialisé sous la dénomination Viton®.

**7.** Cellule de mesure selon l'une des revendications 1 à 6, ***dans laquelle*** ladite enceinte (12) est étanche à un fluide mis sous pression de sorte à permettre l'application d'une pression autour de ladite membrane (15).

**8.** Cellule de mesure selon l'une des revendications 1 à 7, ***dans laquelle*** ladite enceinte (12) intègre des moyens de chauffage (20) de sorte à imposer une température autour de ladite membrane (15).

**9.** Cellule de mesure selon l'une des revendications 1 à 8, ***dans laquelle*** ladite membrane (15) est disposée entre un plateau inférieur (13) et un plateau supérieur (14) disposés au niveau de deux ouvertures de l'enceinte (12), au moins un plateau (13, 14) étant mobile en translation de sorte à compresser ledit matériau durcissable (11) lors de la solidification, lors de la réalisation de tests et/ou de sorte à extraire ledit matériau durcissable (11) de l'enceinte (12).

**10.** Procédé de mesure d'au moins une propriété physique, chimique ou mécanique d'un matériau durcissable (11) lors de la solidification ou après au moyen d'une cellule de mesure selon l'une des revendications 1 à 9, ledit procédé comportant les étapes consistant à :

- mettre lesdits moyens de rigidification (16, 17) dans ledit état de rigidité ;
- verser ledit matériau durcissable (11) sous forme liquide dans ladite membrane (15) ;
- fermer ladite enceinte (12) ;
- solidifier ledit matériau durcissable (11) ;
- lorsque l'état de solidification requis est atteint, mettre lesdits moyens de rigidification (16, 17) dans ledit état de flexibilité ;
- mesurer au moins une propriété physique, chimique ou mécanique dudit matériau durcissable (11) ; et
- extraire ledit matériau durcissable (11) de ladite enceinte (12).

**11.** Procédé de mesure selon la revendication 10, ***dans lequel,*** lorsque ladite cellule de mesure (10a-10b) intègre une enceinte (12) étanche selon la revendication 8, la solidification dudit matériau durcissable (11) est réalisée en appliquant une rampe de pression dans ladite enceinte (12).

**12.** Procédé de mesure selon l'une des revendications 10 ou 11, ***dans lequel,*** lorsque ladite cellule de mesure (10a-10b) intègre des moyens de chauffage (20) selon la revendication 9, la solidification dudit matériau durcissable (11) est réalisée en appliquant une rampe de température dans ladite enceinte (12).

**13.** Procédé de mesure selon l'une des revendications 10 à 12, ***dans lequel,*** lorsque ladite cellule de mesure (10a-10b) intègre un plateau mobile (13, 14) selon la revendication 10, la solidification dudit matériau durcissable (11) est réalisée en appliquant une rampe de contrainte axiale sur ledit matériau durcissable (11).

**14.** Procédé selon l'une des revendications 10 à 13, ***dans lequel,*** l'étape de mesure d'au moins une propriété physique, chimique ou mécanique dudit matériau durcissable (11) consiste à mesurer les déformations axiale et radiale, et/ou la pression de pore, et/ou la vitesse des ondes soniques de compression et de cisaillement, et/ou la résistivité.

15. Procédé selon l'une des revendications 10 à 14, *dans lequel,* suite à l'extraction dudit matériau durcissable (11) de ladite enceinte (12), ledit procédé comporte une étape consistant à analyser ledit matériau durcissable (11).

**Patentansprüche**

1. Messzelle (10a-10b) für mindestens eine physikalische, chemische oder mechanische Eigenschaft eines härtbaren Materials (11), die erwähnte Zelle (10a-10b) umfasst:

   - ein Gehäuse (12);
   - eine flexible Membran (15), die in dem erwähnten Gehäuse (12) so angeordnet ist, dass sie das erwähnte härtbare Material (11) enthält; und
   - Mittel zum Versteifen (16, 17) dieser Membran (15), die dazu konfiguriert sind, zwei alternative Zustände anzunehmen:

     o ein Steifigkeitszustand, bei dem diese Versteifungsmittel (16, 17) der Ausdehnung des härtbaren Materials (11) während des Erstarrens widersteht;
     o und einen flexiblen Zustand, in dem die Versteifungsmittel (16, 17) eine Spannung ausüben, die geringer ist, als die im Steifigkeitszustand ausgeübte, wodurch mindestens eine physikalische, chemische oder mechanische Eigenschaft des härtbaren Materials (11) gemessen und das härtbare Material (11) aus diesem Gehäuse (12) extrahiert werden kann;

   *dadurch gekennzeichnet, dass* die erwähnten Versteifungsmittel aus verschiedenen Metalldrähten (16) bestehen, verbunden durch einen Verschluss (17), der dazu konfiguriert ist, die von diesen Metalldrähten 16) erzeugte Steifigkeit zu verändern.

2. Messzelle nach Anspruch 1, *bei der* die erwähnten Metalldrähte (16) umlaufend, in unterschiedlichen Höhen dieser Membran (15) angeordnet sind, die Enden jedes Metalldrahts (16) sind dabei am Verschluss (17) befestigt.

3. Messzelle nach Anspruch 1 oder 2, *bei der* die Anzahl der Metalldrähte (16) die folgende Gleichung einhält:

$$\frac{L}{2r+e} \leq n \leq \frac{mL}{2r};$$

   - mit $L$ entsprechend der Höhe einer Zylindermembran (15), $r$ dem Radius oder der halben Breite der Metalldrähte, $m$ der Anzahl der Drahtlagen und $e$ der Dicke der Membran.

4. Messzelle nach Anspruch 2, *bei der* das erwähnte Gehäuse (12) mindestens einen radialen Bewegungssensor (18) enthält, der umlaufend um die erwähnte Membran (15), zwischen zwei Metalldrähten (16) angeordnet ist.

5. Messzelle nach einem der Ansprüche 1 bis 4, *bei der* die erwähnte Membran (15) mit einem Elastomerkern ausgeführt wird.

6. Messzelle nach Anspruch 5, *bei der* die erwähnte Membran (15) mit einem Polytetrafluoroethylenkern ausgeführt wird, der unter der Bezeichnung Teflon [R] auf dem Markt ist, oder aus Fluorkarbon- Kautschuk, vermarktet unter der Bezeichnung Viton.

7. Messzelle nach einem der Ansprüche 1 bis 6, *bei der* das erwähnte Gehäuse (12) dicht gegenüber Flüssigkeit unter Druck ist, so dass um diese Membran (15) herum ein Druck ausgeübt werden kann.

8. Messzelle nach einem der Ansprüche 1 bis 7, *bei der* in das erwähnte Gehäuse (12) Heizvorrichtungen (20) integriert sind, so dass um diese Membran (15) herum eine Temperatur angelegt werden kann.

9. Messzelle nach einem der Ansprüche 1 bis 8, bei der diese Membran (15) zwischen einer unteren Platte (13) und einer oberen Platte (14) angeordnet ist, die sich in Höhe der beiden Öffnungen des Gehäuses (12), befinden, mindestens eine Platte (13, 14) ist quer verschiebbar, so dass das erwähnte härtbare Material (11) bei der Aushärtung und/ oder der Durchführung von Tests zusammengepresst wird und/ oder um dieses härtbare Material (11) aus dem Gehäuse (12) zu extrahieren.

10. Verfahren zur Messung mindestens einer physikalischen, chemischen oder mechanischen Eigenschaft eines härtbaren Materials (11) bei der Aushärtung oder danach, mittels einer Messzelle nach einem der Ansprüche 1 bis 9, dieses Verfahren umfasst die folgenden Schritte:

- diese Versteifungsmittel (16, 17) in den erwähnten Steifigkeitszustand bringen;
- das erwähnte härtbare Material (11) in flüssiger Form in die erwähnte Membran (15) gießen;
- das erwähnte Gehäuse (12) schließen;
- das erwähnte härtbare Material (11) aushärten;
- wenn der geforderte Aushärtungszustand erreicht ist, die erwähnen Versteifungsmittel (16, 17) in den erwähnten flexiblen Zustand bringen;
- mindestens eine physikalische, chemische oder mechanische Eigenschaft dieses härtbaren Materials (11) messen; und
- dieses härtbare Material (11) aus diesem Gehäuse (12) extrahieren.

11. Messverfahren nach Anspruch 10, *bei dem,* wenn die erwähnte Messzelle (10a-10b) ein dichtes Gehäuse (12) nach Anspruch 8 enthält, die Aushärtung dieses härtbaren Materials (11) durch Anwendung einer Druckrampe in diesem Gehäuse (12) erfolgt.

12. Messverfahren nach einem der Ansprüche 10 oder 11, *bei dem,* wenn die erwähnte Messzelle (10a- 10b) Heizvorrichtungen (20) nach Anspruch 9 enthält, die Aushärtung dieses härtbaren Materials (11) durch Anwendung einer Temperaturrampe in diesem Gehäuse (12) erfolgt.

13. Messverfahren nach einem der Ansprüche 10 bis 12, *bei dem,* wenn die erwähnte Messzelle (10a-10b) eine mobile Platte (13, 14) nach Anspruch 10 enthält, die Aushärtung dieses härtbaren Materials (11) durch Anwendung einer axialen Spannungsrampe auf dieses härtbaren Materials (11) erfolgt.

14. Verfahren nach einem der Ansprüche 10 bis 13, *bei dem* der Schritt der Messung mindestens einer physikalischen, chemischen oder mechanischen Eigenschaft dieses härtbaren Materials (11) darin besteht, die axialen und radialen Verformungen, und/ oder den Porendruck und/ oder die Geschwindigkeit der Kompressions- und Scher- Schallwellen und/ oder den Widerstand zu messen.

15. Verfahren nach einem der Ansprüche 10 bis 14, *bei dem* nach der Extraktion dieses härtbaren Materials (11) aus dem Gehäuse (12), dieses Verfahren einen Schritt enthält, der in einer Analyse dieses härtbaren Materials (11) besteht.

## Claims

1. A cell for measuring (10a-10b) at least one physical, chemical, or mechanical property of a hardenable material (11), said cell (10a-10b) comprising:

- an enclosure (12);
- a flexible membrane (15) disposed in said enclosure (12) in such a way as to contain said hardenable material (11); and
- means for stiffening (16, 17) said membrane (15) configured to assume two alternative states:

  ∘ a stiffness state, in which said stiffening means (16, 17) resist the expansion of said hardenable material (11) during solidification; and
  ∘ a flexible state, in which said stiffening means (16, 17) exert a stress that is less than that exerted in the stiffness state, allowing at least one physical, chemical, or mechanical property of said hardenable material (11) to be measured and said hardenable material (11) to be extracted from said enclosure (12);

*characterized in that* said stiffening means are produced by a set of metal wires (16) connected to a clasp (17) configured to modify the stiffness exerted by said metal wires (16).

2. A measurement cell according to claim 1, **wherein** said metal wires (16) are disposed circumferentially at multiple heights of said membrane (15), the ends of each metal wire (16) being fastened to said clasp (17).

3. The measurement cell according to claim 1 or 2, **wherein** the number $n$ of metal wires (16) meets the following condition:

$$\frac{L}{2r+e} \leq n \leq \frac{mL}{2r};$$

where $L$ corresponding to the height of a cylindrical membrane (15), $r$ to the radius or half-width of the metal wires, $m$ to the number of wire layers, and $e$ to the thickness of the membrane.

4. The measurement cell according to claim 2, **wherein** said enclosure (12) comprises at least one radial motion sensor (18) disposed circumferentially around said membrane (15) between two metal wires (16).

5. The measurement cell according to one of claims 1 to 4, **wherein** said membrane (15) is made having an elastomer core.

6. The measurement cell according to claim 5, **wherein** said membrane (15) is made having a core of polytetrafluoroethylene, such as the kind sold under the name Teflon®, or of fluorocarbon rubber, such as the kind sold under the name Viton®.

7. The measurement cell according to one of claims 1 to 6, **wherein** said enclosure (12) is resistant to leaks of a pressurized fluid, so as to allow pressure to be applied around said membrane (15).

8. The measurement cell according to one of claims 1 to 7, **wherein** said enclosure (12) incorporates heating means (20) so as to apply a temperature around said membrane (15).

9. The measurement cell according to one of claims 1 to 8, **wherein** said membrane (15) is disposed between a lower plate (13) and an upper plate (14) disposed at two openings in the enclosure (12), at least one plate (13, 14) being movable in translation so as to compress said hardenable material (11) during solidification, when conducting tests and/or to extract said hardenable material (11) from the enclosure (12).

10. A method for measuring at least one physical, chemical, or mechanical property of a hardenable material (11) during solidification or afterward by means of a measurement cell according to one of claims 1 to 9, said method comprising the steps of:

    - putting said stiffening means (16, 17) into said stiffness state;
    - pouring said hardenable material (11) in liquid form into said membrane (15);
    - closing said enclosure (12);
    - solidifying said hardenable material (11);
    - when the required solidification state is achieved, putting said stiffening means (16, 17) into said flexible state;
    - measuring at least one physical, chemical, or mechanical property of said hardenable material (11); and
    - extracting said hardenable material (11) from said enclosure (12).

11. The measurement method according to claim 10, **wherein,** when said measurement cell (10a-10b) incorporates a leak-tight enclosure (12) according to claim 8, said hardenable material (11) is solidified by applying a pressure ramp within said enclosure (12).

12. The measurement method according to one of claims 10 or 11, **wherein,** when said measurement cell (10a-10b) incorporates heating means (20) according to claim 9, said hardenable material (11) is solidified by applying a temperature ramp within said enclosure (12).

13. A measurement method according to one of the claims 10 to 12, **wherein,** when said measurement cell (10a-10b) incorporates a moving plate (13, 14) according to claim 10, said hardenable material (11) is solidified by applying an axial stress ramp on said hardenable material (11).

**14.** The method according to one of claims 10 to 13, *wherein,* the step of measuring at least one physical, chemical, or mechanical property of said hardenable material (11) involves measuring the axial and radial deformations, and/or the pore pressure, and/or the velocity of the sonic compressional and shear waves, and/or the resistivity.

**15.** The method according to one of claims 10 to 14, *wherein,* following the extraction of said hardenable material (11) from said enclosure (12), said method comprises a step consisting of analyzing said hardenable material (11).

EP 3 559 661 B1

Fig. 1

14

Fig. 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2012049620 A